(19) Europäisches Patentamt
European Patent Office
Office européen des brevets

(11) Numéro de publication: **0 378 941 A2**

(12) **DEMANDE DE BREVET EUROPEEN**

(21) Numéro de dépôt: **89403540.1**

(51) Int. Cl.5: **C07H 19/073, A61K 31/70**

(22) Date de dépôt: **19.12.89**

Une requête en rectification du texte a été présentée conformément à la règle 88 CBE. Il est statué sur cette requête au cours de la procedure engagée devant la division d'examen (Directives relatives à l'examen pratiqué à l'OEB, A-V, 2.2).

Revendications pour l'Etat contractant suivant: GR.

(30) Priorité: **20.12.88 FR 8816822**

(43) Date de publication de la demande: **25.07.90 Bulletin 90/30**

(84) Etats contractants désignés: **AT BE CH DE FR GB GR IT LI LU NL SE**

(71) Demandeur: **INSTITUT MERIEUX**
**58 avenue Leclerc**
**F-69342 Lyon Cedex 07(FR)**

(72) Inventeur: **Ronco, Gino Lino**
**11 Bd Pont Noyelles**
**F-80000 Amiens(FR)**
Inventeur: **Villa, Pierre Joseph**
**43 Rue Maréchal de Castries**
**F-80000 Amiens(FR)**
Inventeur: **Vilie, Guy André**
**15 Rue Charles Friedel**
**F-75020 Paris(FR)**

(74) Mandataire: **Bernasconi, Jean et al**
**CABINET LEMOINE ET BERNASCONI 13, Boulevard des Batignolles**
**F-75008 Paris(FR)**

(54) **Nouveaux dérivés de désoxy-2 ribonucléosides et leurs applications.**

(57) Les nouveaux dérivés de désoxy-2 ribonucléosides conformes à l'invention sont caractérisés en ce qu'ils répondent à la formule générale

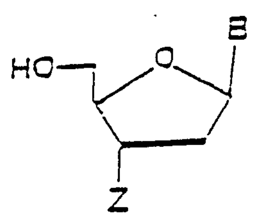

(I)

dans laquelle B désigne une base pyrimidique pouvant être la thymine ou l'uracyle ou un dérivé équivalent
A- C -X
  ||
  Y
ou encore un groupement $N(CN)_2$ ou $N(CONH_2)_2$ où A représente un radical alkyle saturé ou non, substitué ou non par des groupements carbonés ou des hétéroatomes, ou un groupe aminé primaire, secondaire ou tertiaire, ou encore un cycle insaturé ou non, comportant ou non des hétéroatomes, X représente un atome d'azote, d'oxygène ou de soufre ou encore de sélénium, lié au carbone du cycle glucidique, et Y représente un atome d'azote, d'oxygène, de soufre ou de sélénium, à l'exclusion des dichlorhydrates de O-[-ω-[N-[ω-(diéthylamino) alkyl] diéthylammonio]-acyl-]-3′ thymidine, des esters du N,N′-di-n-alkyl-diazabicyclo-1,4 octane [2.2.2.] et du O-[Phénoxy(thiocarbonyl)]-3′ -desoxy-2′-thymidine.

EP 0 378 941 A2

## Nouveaux dérivés de désoxy-2 ribonucléosides et leurs applications.

La présente invention a trait à de nouveaux dérivés de désoxy-2 ribonucléosides.

Elle concerne également l'application de ces dérivés à titre de médicaments.

Il est connu que certains ribonucléosides modifiés possèdent d'intéressantes propriétés biologiques et qu'ils sont notamment utilisés comme agents antibiotiques, antiviraux et antitumoraux.

L'activité antivirale de certains dérivés de désoxy-2 ribonucléosides tels que l'azido-3′ désoxy-3′ thymidine (AZT) et l'isothiocyanato-3′ désoxy-3′ thymidine (ITCT) a été attribuée à la présence d'atomes comportant au moins un doublet libre en position 1 et 3 sur la chaîne du substituant en 3′.

L'un des buts de la présente invention est de fournir de nouveaux dérivés de désoxy-2 ribonucléosides comportant une chaîne latérale en position 3′, ladite chaîne étant caractérisée en ce qu'elle comporte au moins trois atomes, le premier et le troisième possédant au moins un doublet électronique libre.

Il est possible de préparer ces nouveaux dérivés par un procédé d'une mise en oeuvre simple, en conduisant à de bons rendements.

Un autre but encore de l'invention est de fournir des dérivés de désoxy-2 ribose présentant une grande activité biologique, rendant ces dérivés d'une application particulièrement intéressante pour la préparation de médicaments antibiotiques, antiviraux, antitumoraux et immunomodulateurs.

Les nouveaux dérivés de désoxy-2 ribonucléosides conformes à l'invention sont caractérisés en ce qu'ils répondent à la formule générale

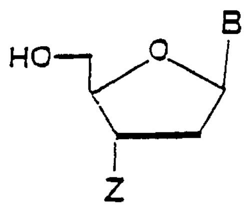

(I)

dans laquelle B désigne une base pyrimidique pouvant être la thymine ou l'uracyle ou un dérivé équivalent

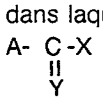

ou encore un groupement $N(CN)_2$ ou $N(CONH_2)_2$ où A représente un radical alkyle saturé ou non, substitué ou non par des groupements carbonés ou des hétéroatomes, ou un groupe aminé primaire, secondaire ou tertiaire, ou encore un cycle insaturé ou non, comportant ou non des hétéroatomes, X représente un atome d'azote, d'oxygène ou de soufre ou encore de sélénium, lié au carbone du cycle glucidique, et Y représente un atome d'azote, d'oxygène, de soufre ou de sélénium, à l'exclusion des dichlorhydrates de O-[-ω-[N-[ω-(diéthylamino) alkyl] diéthylammonio]-acyl-]-3′ thymidine, des esters du N,N'-di-n-alkyl-diazabicyclo-1,4 octane [2.2.2.] et du O-[Phénoxy(thiocarbonyl)]-3′ -desoxy-2′-thymidine.

Les composés préférés conformes à l'invention sont ceux répondant à la formule générale

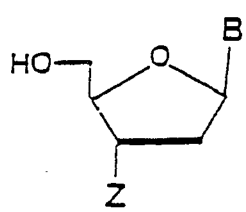

dans laquelle B a la signification précitée et Z désigne un groupement

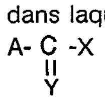

où X et Y ont la signification précitée et A représente :

- un radical alkyle inférieur contenant 1 à 8 atomes de carbone, substitué ou non par plusieurs atomes d'halogène, et plus particulièrement le fluor,
- ou encore un radical diméthylamino, diéthylamino, dipropylamino, diisopropylamino imidazolo et morpholino.

D'autres composés préférés conformes à l'invention sont ceux répondant à la formule générale

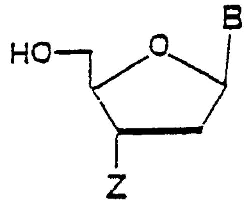

dans laquelle B a la signification précitée et Z désigne le groupement $N(CN)_2$ ou $N(CONH_2)_2$.

Parmi les dérivés de désoxy-2 ribonucléosides conformes à l'inv ention, plus particulièrement préférés, on peut citer les composés suivants :

- la O-butanoyl-3' thymidine

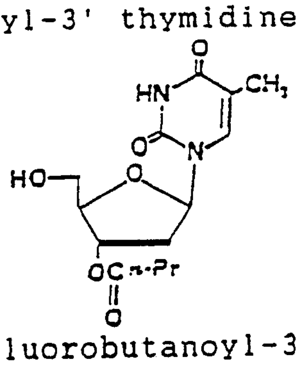

- la O-heptafluorobutanoyl-3' thymidine

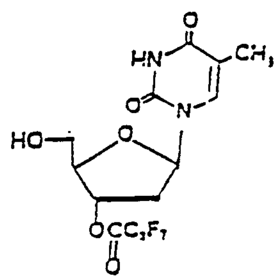

- la S-(N,N-diéthylthiocarbamoyl)-3' désoxy-3' thio-3' thymidine

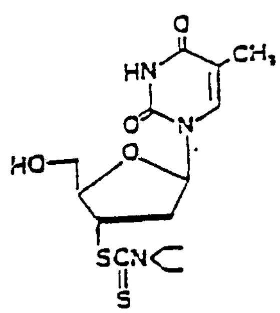

la O-(N,N-diméthylthiocarbamoyl)-3' thymidine

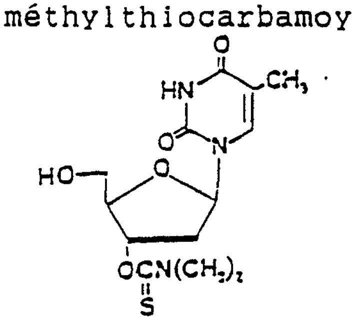

- la dicyanamido-3' désoxy-3' thymidine

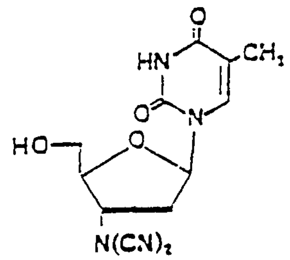

- la [N amido-1] uréido-3' désoxy-3' thymidine

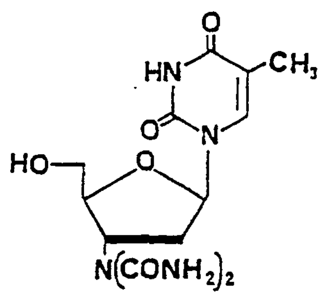

- la O-(imidazolothiocarbonyl)-3' thymidine

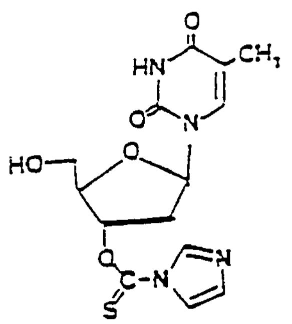

- la O-(morpholinothiocarbonyl)-3' thymidine

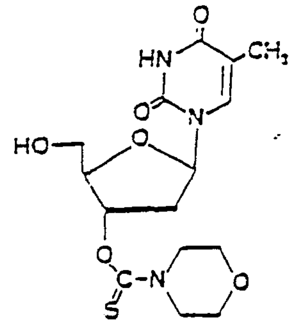

Ces composés peuvent être préparés par un procédé caractérisé en ce que partant d'un composé de formule générale

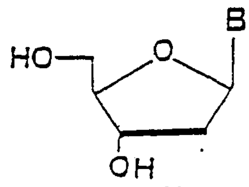

(II)

dans llaquelle B désigne une base pyrimidique qui peut être la thymine ou l'uracile,
il comporte les étapes :

- a) de protection du site OH primaire en position 5′;

b) d'estérification du site OH en position 3′ par un chlorure ou un anhydride d'acide carboxylique ou sulfonique;

- c) de substitution par un anion Z de type

[A- C -X]$^-$
||
Y

ou $[N(CN)_2]^-$

où A représente un radical alkyle saturé ou non, substitué ou non, par des groupements carbonés ou des hétéroatomes ou un groupe amine primaire, secondaire ou tertiaire ou encore un cycle insaturé ou non, comportant ou non des hétéroatomes, X représente un atome d'azote, d'oxygène, de soufre ou de sélénium, dans le cas des esters sulfoniques ;
et Y représente un atome d'azote, d'oxygène, de soufre ou de sélenium.

- d) de déprotection du site OH préalablement bloqué.

La protection du site OH en position 5′ peut être réalisée, par exemple, en faisant réagir le composé de formule (II) avec un composé de formule $Ar_3C$ Cl (Ar désignant un radical aryle) par la méthode décrite par H.R. MUNSON dans "Synthetic Procedures in Nucleic Acid Chemistry "Vol. 1 ; Ed. Wiley ; Londres". On peut également réaliser la protection de ce site OH en faisant réagir le composé de formule (II) avec un composé de formule $R_3Si$ Cl selon la méthode décrite par K.K. OGILVIE et D.J. IWACHA dans Tetrahedron Letters, 1973, 4 31. La réaction avec $Ar_3$ C Cl ou $R_3Si$ Cl s'effectue en présence d'une base dans un solvant approprié.

Dans le cas d'une protection par $Ar_3$ C Cl, les trois groupements aryles peuvent être identiques ou différents et choisis parmi les radicaux phényle, p-méthoxyphényle, p-toluyle.

Dans le cas d'une protection par $R_3SiCl$, les trois groupements (R) peuvent être identiques ou différents et choisis parmi les radicaux méthyle, t-butyle, phényle, naphtyle.

Le solvant peut être choisi parmi les solvants organiques suivants : l'hexaméthylphosphoramide (HMPA), la diméthylformamide (DMF), le diméthylsulfoxyde (DMSO), la pyridine, le dioxanne, le tétrahydrofuranne (THF), le dichlorométhane ($CH_2$ $Cl_2$). Ces solvants sont utilisés seuls ou en mélange, ou encore associés avec d'autre solvants organiques.

La base peut être choisie parmi l'une des bases organiques suivantes : pyridine, diaza-1,8-bicyclo [5,4,0] undécène-7 (DBU), amines tertiaires.

Lors de l'étape b), l'estérification du site OH en position 3′ est réalisée par un chlorure d'acide de formule

R'-$SO_2$-Cl
ou
R''-CO-Cl
ou
R''-CS-Cl
ou encore par un anhydride d'acide de formule

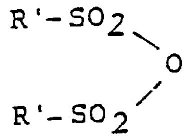

ou

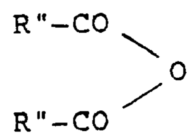

dans laquelle R' représente un radical méthyle, trifluorométhyle, phényle, p-toluyle, p-bromophényle, imidazole et R'' représente un radical alkyle saturé ou non, substitué ou non par des groupements carbonés ou des hétéroatomes ou encore un cycle insaturé ou non, comportant ou non des hétéroatomes.

Lorsque l'estérification est effectuée par des chlorures ou des anhydrides d'acides sulfoniques, le groupement partant sulfonate est déplacé par un anion Z de type

$[A\text{-} \underset{\underset{Y}{\|}}{C} \text{-}X]^-$

ou de type $[N(CN)_2]^-$ où A a la même signification que le groupe R'' indiqué plus haut, X représentant un atome d'azote, d'oxygène ou de soufre ou encore de sélénium et Y un atome d'azote, d'oxygène, de soufre ou de sélénium.

L'anion est de préférence introduit sous forme de sel dans le milieu approprié. Le cation associé peut être avantageusement constitué par un métal alcalin tel que lithium, sodium ou potassium.

Ce sel est ajouté au milieu dans un rapport molaire généralement compris entre 1,1 et 9, et de préférence voisin de 5 par rapport à l'intermédiaire obtenu à la fin de l'étape (b).

Dans une dernière étape (étape (d)) on procède à la déprotection du site 5' bloqué.

Dans le cas où ce site est protégé par $Ar_3C$, la déprotection peut être effectuée en présente d'un acide organique ou minéral dans un solvant approprié.

Dans le cas où le site 5' est protégé par $R_3Si$, la déprotection peut être effectuée en présence d'un acide organique ou minéral, ou en présence d'ions fluorure dans un solvant approprié.

L'acide minéral peut être choisi parmi l'acide chlorhydrique, l'acide bromhydrique, l'acide fluoroborique, le chlorure d'étain, le chlorure de titane.

L'acide organique peut être choisi parmi l'acide acétique, l'acide trifluoroacétique, l'acide p-toluénesulfonique.

Le cation associé à l'ion fluorure peut être choisi parmi le potassium, le sodium ou encore les ammoniums quaternaires.

Le solvant peut être l'eau, un alcool, le tétrahydrofuranne, ou un mélange de ces solvants ou encore l'un de ces solvants associé à d'autres solvants organiques.

La présente invention concerne également l'application des dérivés de désoxy-2 ribonucléosides de formule (I) à la préparation de médiaments.

Elle a aussi pour objet des compositions pharmaceutiques contenant un dérivé de formule (I) éventuellement en mélange avec des diluants ou véhicules pharmaceutiquement acceptables. Ces compositions se présentent sous une forme destinée à l'administration par voie entérale, de préférence orale, par exemple sous forme de comprimés ou de capsules,ou destinée à l'administration par voie parentérale, par exemple sous forme de solutions ou de préparations injectables stériles.

Les compositions conformes à l'invention sont utiles pour le traitement et la prévention des maladies virales ainsi que pour les traitements antitumoraux. Les nouveaux dérivés de désoxy-2 ribonucléosides conformes à l'invention trouvent également une application intéressante comme médicaments antibiotiques et immunomodulateurs.

Il va être donné, ci-dessous, quelques exemples de mise en oeuvre de l'invention, à titre illustratif et non limitatif.

Exemple 1 :

Synthèse de la O-butanoyl-3′ thymidine.

Cette synthèse est réalisée selon le schéma réactionnel suivant :

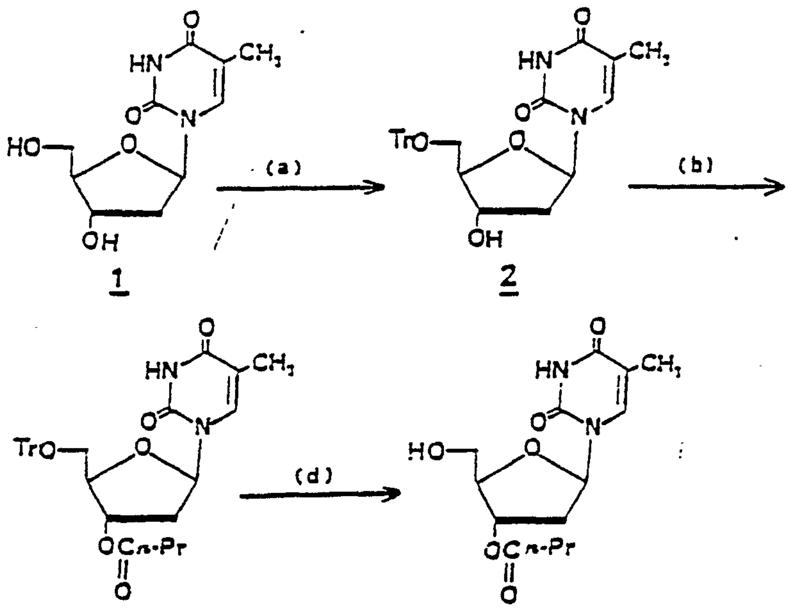


1

2

- Etape (a) préparation de la O-trityl-5′ thymidine:

2,4 g (10 mmol) de thymidine et 3,2 g (11,5 mmol) de chlorure de trityle sont dissous dans 50 ml de pyridine anhydre puis le mélange est porté à 100 $^\circ$C (bain d'huile) et laissé à reflux pendant une heure sous agitation. La solution refroidie est versée dans de l'eau froide en agitant ; le précipité est essoré puis lavé à l'eau. Le produit brut est conservé 24 h dans un dessicateur. Il est ensuite lavé à l'éther éthylique afin d'éliminer toute trace de chlorure de trityle et l'on obtient 3,77 g d'un solide blanc (rendement 78 %).
Caractéristiques physiques : F = 135 $^\circ$C ; $[\alpha]_D^{20}$ = 21,0 $^\circ$ (c = 0,5 ; acétone).

- Etape (b) préparation de la O-butanoyl-3′ O-trityl-5′ thymidine.

Un mélange de 2 mmol (0,97 g) de O-trityl-5′ thymidine et 2,5 mmol (0,4 g) d'anhydride butyrique dans 10 ml de pyridine anhydre est agité à température ambiante. On suit l'avancement de la réaction par CCM sur plaque de silice en prenant l'acétate d'éthyle comme éluant.

Lorsque la réaction est terminée, on neutralise par une solution de $NaHCO_3$, on extrait à l'éther éthylique, puis on décante la phase organique que l'on sèche sur sulfate de sodium anhydre. Après filtration et évaporation du solvant sous pression réduite, on obtient 0,96 g d'un solide blanc (rendement 87 %).
Caractéristiques physiques F = 92 $^\circ$C ; $[\alpha]_D^{20}$ = 16,0 $^\circ$ (c = 1 ; acétone))

- Etape (d) préparation de la O-butanoyl-3′ thymidine :

Dans un ballon de 100 ml, on place 0,5 g (0,9 mmol) du composé 3 et 6 ml d'acide acétique à 80 % et le mélange est chauffé à 100 $^\circ$C sous agitation. Après 12 mn on évapore l'acide acétique restant sous pression réduite. Le compose est séparé du mélange par chromatographie sur colonne 1 : 1 (V/V). On obtient 188 mg de produit pur (rendement 67 %).
Caractéristiques physiques : F = 146 - 148 $^\circ$C ; $[\alpha]_D^{20}$ = - 7,5 $^\circ$ (c = 1 ; $CHCl_3$).

Exemple 2

Synthèse de la O-heptafluorobutanoyl-3′ thymidine

La synthèse est réalisée selon le schéma réactionnel suivant :

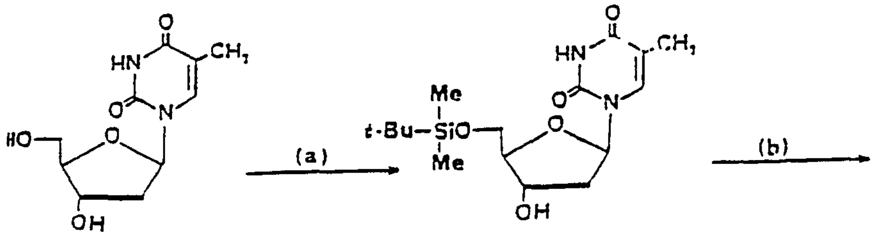


1 2′

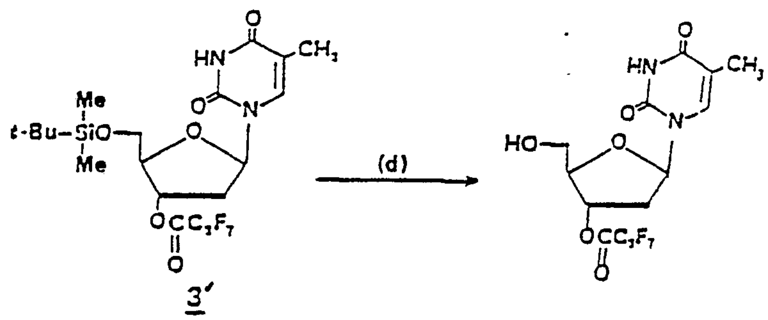


3′

- Etape (a) préparation de la O-(t-butyldiméthylsilyl)-5′ thymidine :

Une solution contenant 4,21 g (10 mmol) de thymidine, 2,75 g(11 mmol) de t-butyldiméthylchlorosilane (TBDMSCl) et 1,7 g (25 mmol) d'imidazole dans 10 ml de DMF est maintenue à 30° C pendant 24 heures. On obtient un mélange de produits mono et disilylé (80/20) que l'on sépare sur colonne de silice en utilisant l'éther éthylique comme éluant. On isole 2,13 g de produit pur (rendement = 60 %).
Caractéristiques physiques : F = 193-194° C.

- Etape (b) préparation de la O-(-t-butyldiméthylsilyl)-5′ O-heptafluorobutanoyl-3′ thymidine :

A une solution renfermant 2 g (5,6 mmol) de 2′ et 1,1 ml de triéthylamine dans 20 ml de chloroforme anhydre, on ajoute goutte à goutte 1,4 g (6 mmol) de chlorure d'heptafluorobutanoyle, fraîchement préparé, en solution dans 3 ml de chloroforme. Lorsque l'addition est terminée, le mélange réactionnel est maintenu 3 heures à température ambiante. A la fin de la réaction, on évapore le chloroforme puis le résidu obtenu est repris à l'éther et lavé avec une solution saturée de NaCl. Après séchage de la phase organique sur $MgSO_4$ et évaporation du solvant sous pression réduite, le produit brut obtenu est purifié par chromatographie en phase liquide sur colonne de gel de silice en utilisant un gradient éther éthylique-pentane comme éluant. On isole 2,5 g de produit pur (rendement 81 %).

- Etape (d) préparation de la O-heptafluorobutanoyl-3′ thymidine :

On place 2,2 g (4 mmol) de 3′ dans 6 ml de solution 1 M de fluorure de triéthylammonium dans la pyridine pendant 30 mn. On ajoute ensuite 2,5 ml de solution saturée d'hydrogénocarbonate de sodium puis on extrait à l'éther éthylique. Après séchage de la phase organique sur $MgSO_4$ anhydre et évaporation du solvant sous pression réduite, on purifie le produit brut restant par chromatographie liquide sur colonne de gel de silice en utilisant l'acétate d'éthyle comme éluant. On obtient 1,54 g de produit pur (rendement 88 %).

Exemple 3

Préparation de la désoxy-3′-S-[N,N diméthylthiocarbamoyl]-3′ thio-3′ thymidine.

Le produit est préparé selon la séquence suivante :

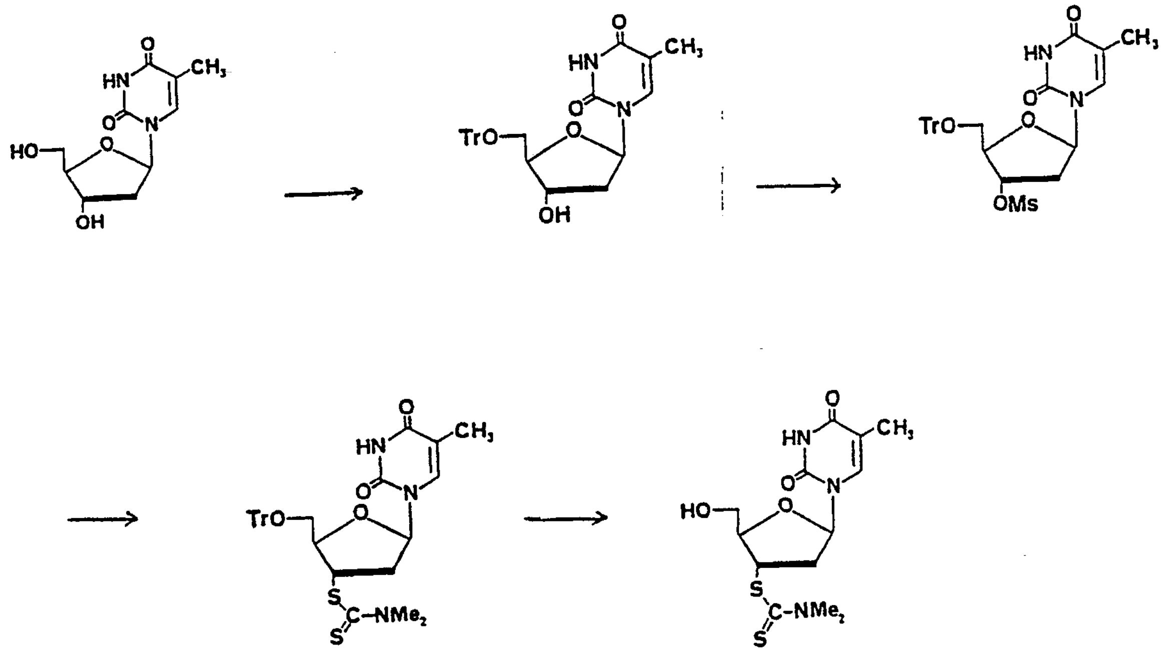

L'étape (a) est réalisée suivant le protocole décrit dans l'exemple 1.

-Etape (b) préparation de la O-méthanesulfonyl-3′ O-trityl-5′ thymidine :

On dissout 2 mmol (0,96 g) de O-trityl-5′ thymidine dans 5 ml de pyridine et on refroidit à -10° C. On ajoute alors 4,4 mmol (0,5 g) de chlorure de méthanesulfonyle en agitant fortement pendant l'introduction. Le mélange est alors conservé 24 heures à 0° C. Lorsque la réaction est terminée (contrôle par CCM), la solution est versée dans de l'eau froide et agitée pendant 40 mn. Après séchage de la phase organique sur $MgSO_4$ et évaporation du solvant sous pression réduite, on obtient 1,09 g d'un solide de couleur blanche (rendement 97 %).

Caractéristiques physiques : F = 149 ° C ; $[\alpha]$-$_D^{20}$ = 20° (c=2 ; acétone).

- Etape (c) préparation de la désoxy-3′-S-[N,N diméthyl thiocarbamoyl]-3′ thio-3′ thymidine.

On dissout 1 mmol (0,56 g) de O-méthanesulfonyl-3′ O-trityl-5′, thymidine et 5 mmol de diméthyl dithiocarbamate de potassium dans 16 ml de DMF puis on agite à une température de 100° C. On suit l'avancement de la réaction par CCM (éluant acétate d' ethyl) . Lorsque la réaction est terminée, on ajoute de l'eau à la solution et on agite fortement. On filtre le précipité formé et on le laisse 24 heures dans un dessicateur. On obtient 510 mg de produit pur (rendement 87 %).

Caractéristiques physiques F = 82° C ; $[\alpha]_D^{20}$ = 11,0° (c=0,3 acétate d'éthyle).
L'étape (d) est réalisée suivant le protocole décrit dans l'exemple 1. Le rendement de cette étape est de 70%.
Caractéristiques physiques : F = 95° C ; $[\alpha]_D^{20}$ = 17,4° (c = 0,4 ; acétate d'éhyle) ; IR ($cm^{-1}$: $\nu_{OH}$ = 3300, $\nu_{C=S}$=1690.

Exemple 4

Préparation de la S-(N,N-diéthylthiocarbamoyl)-3′ désoxy-3′ thio-3′ thymidine :

Le produit est préparé selon la séquence suivante

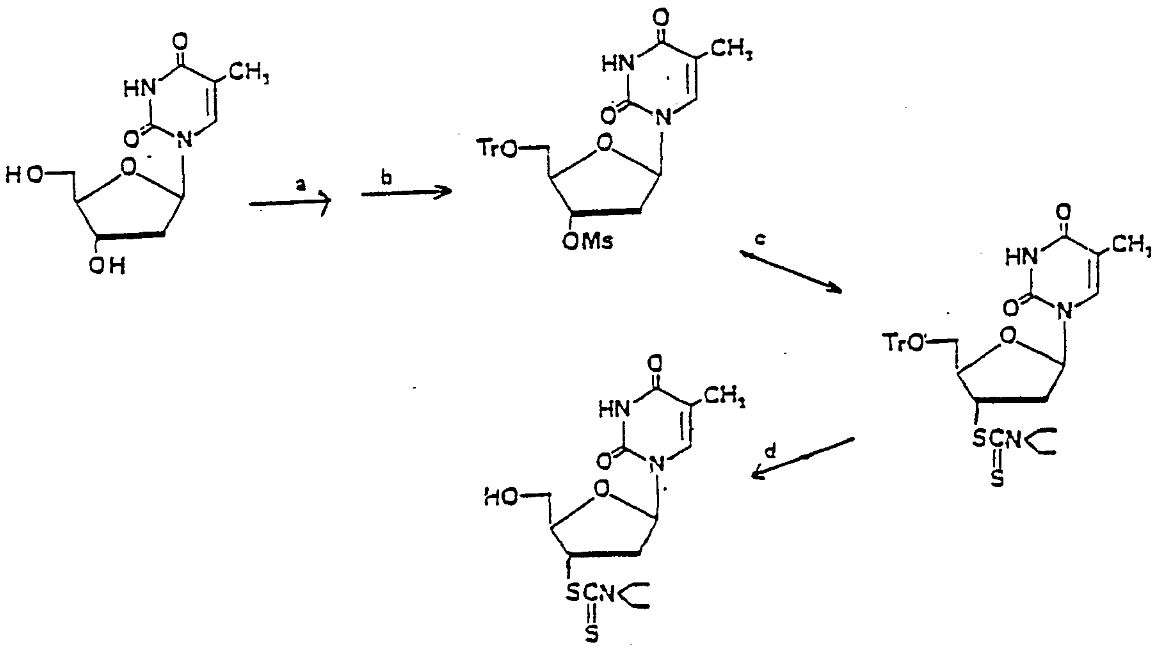


L'étape (a) est réalisée selon le protocole décrit dans l'exemple 1.

L'étape (b) est réalisée suivant le protocole décrit dans l'exemple 3.

L'étape (c) est réalisée dans les mêmes conditions que dans l'exemple 3 en remplaçant le thioacétate de potassium par du N,N-diéthyl dithiocarbamate de potassium dans les mêmes proportions. Le rendement de cette étape est de 76 %.

Caractéristiques physiques : F = 69-70 °C ; $[\alpha]_D^{20}$ = 2,73° (c = 2 acétone).

L'étape (d) est réalisée suivant le protocole décrit dans l'exemple 1. Le rendement de cette étape est de 70 %.

Caractéristiques physiques : $[\alpha]_D^{20}$ = 24,8° (c = 1 ; acétone) ; IR ($cm^{-1}$) : $\nu_{OH}$ = 3420, $\nu_{C=S}$ = 1690.

Préparation de la désoxy-3′ [dicyanamido]-3′ thymidine.

La synthèse est réalisée selon le schéma réactionnel suivant :

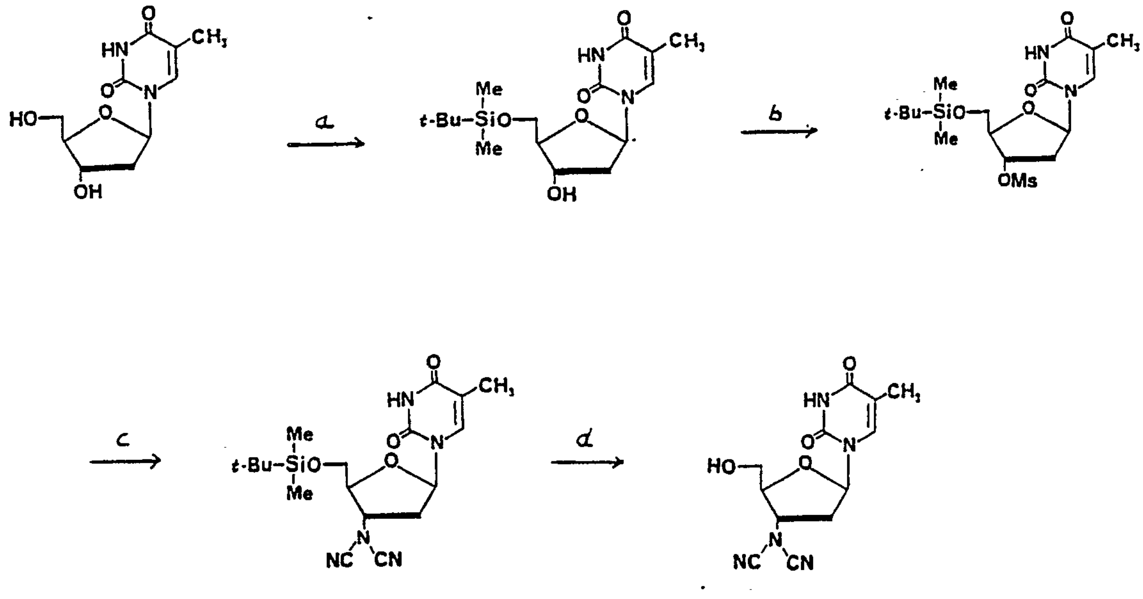


Etape (a) réalisée selon le protocole décrit dans l'exemple 2.

- Etape (b) préparation de la O tert-butyl diméthyl-silyl-5′-O-[méthanesulfonyl]-3′ thymidine :

On dissout 3,9 mmol (1,4 g) de O tert-butyl diméthylsilyl-5′ thymidine dans 15 ml de pyridine et on refroidit le mélange à -10° C. On ajoute alors 5,7 mmol de chlorure de méthanesulfonyle en agitant. L'avancement de la réaction est suivi par CCM en utilisant l'acétate d'éthyle comme éluant ; lorsque la réaction est terminée, le mélange réactionnel est versé dans de l'eau glacée en agitant. Le précipité formé est lavé à l'eau puis séché au dessicateur. Rendement 85 % (1,45 g). Caractéristiques physiquse : F = 140° C ; $[\alpha]_D^{20}$ = 8,1° (c = 1, chloroforme).

- Etape (c) préparation de la O tert-butyl diméthylsilyl-5′ désoxy-3′ dicyanamido-3′ thymidine :

On dissout 3,2 mmol (1,4 g) du composé obtenu à l'étape (b) dans 40 ml de DMF puis on ajoute 11,2 mmol (1,0 g) de dicyanamidure de sodium, le mélange est agité à 110° C pendant 72 h puis la solution est versée dans l'eau froide et extraite à l'acétate d'éthyle. Après lavage de la phase organique à l'eau, séchage sur $Na_2SO_4$, filtration et évaporation du solvant, on obtient 0,88 g d'un solide jaune pâle, rendement 68%. Caractéristiques physiques : F = 148° C; $[\alpha]_D^{20}$ = 53,7°, c = 0,7, méthanol ; IR($cm^{-1}$) $\nu_{C1\equiv N}$ 2250, 2230, 2170, 2140.

L'étape (d) est réalisée selon le protocole décrit dans l'exemple 2. Le rendement est de 53 %. Caractéristiques physiques : F = 110° C ; $[\alpha]_D^{20}$ = 8,1° (c = 0,7, eau). IR ($cm^{-1}$) $\nu_{C\equiv N}$ = 2230, 2190, 2170.

Les composés selon l'invention s'avèrent posséder des propriétés thérapeutiques intéressantes, notamment pour le traitement et la prévention de maladies virales, y compris le virus HIV, notamment en ce qui concerne les infections multiples associées au virus HIV.

## Revendications

1. Dérivé de désoxy-2 ribonucléoside caractérisé en ce qu'il répond à la formule générale

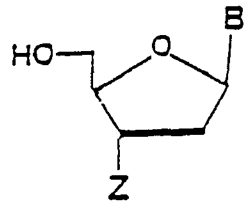

(I)

dans laquelle B désigne une base pyrimidique telle que la thymine ou l'uracyle ou un dérivé équivalent et Z et un groupement

$A-\underset{\underset{Y}{\|}}{C}-X$

ou encore un groupement $N(CN)_2$ ou $N(CONH_2)_2$ où A représente un radical alkyle saturé ou non, substitué ou non par des groupements carbonés ou des hétéroatomes, ou un groupe aminé primaire, secondaire ou tertiaire, ou encore un cycle insaturé ou non, comportant ou non des hétéroatomes, X représente un atome d'azote, d'oxygène ou de soufre ou encore de sélénium, lié au carbone du cycle glucidique, et Y représente un atome d'azote, d'oxygène, de soufre ou de sélénium, à l'exclusion des dichlorhydrates de O-[-ω-[N-[ω-(diéthylamino) alkyl] diéthylammonio]-acyl-]-3' thymidine, des esters du N,N'-di-n-alkyl-diazabicyclo-1,4 octane [2.2.2.] et du O-[Phénoxy(thiocarbonyl)]-3' -desoxy-2'-thymidine.

2. Dérivé de désoxy-2 ribonucléoside caractérisé en ce qu'il répond à la formule générale donnée dans la revendication 1, dans laquelle B a la signification précitée et Z représente un groupement

$A-\underset{\underset{Y}{\|}}{C}-X$

où X et Y ont la signification précitée et A représente un radical alkyle inférieur contenant 1 à 8 atomes de carbone substitué ou non par un ou plusieurs atomes d'halogène, et plus particulièrement par des atomes de fluor.

3. Dérivé de désoxy-2 ribonucléoside selon la revendication 1, caractérisé en ce qu'il répond à la formule générale donnée dans la revendication 1, dans laquelle B a la signification précitée et Z représente un groupement

$A-\underset{\underset{Y}{\|}}{C}-X$

où X et Y ont la signification précitée et A représente un groupement diméthylamino, diéthylamino, isopropylamino, diisopropylamino, morpholino et imidazolo.

4. Dérivé de désoxy-2 ribonucléoside selon la revendication 1, caractérisé en ce qu'il répond à la formule générale donnée dans la revendication 1 dans laquelle B a la signification précitée et Z représente un groupement $N \equiv C-X$ où X représente l'azote.

5. Dérivé de désoxy-2 ribonucléoside selon la revendication 2, caractérisé en ce qu'il est constitué par la O-butanoyl-3' thymidine.

6. Dérivé de désoxy-2 ribonucléoside selon la revendication 2, caractérise en ce qu'il est constitué par l'O-heptafluorobutanoyl-3' thymidine.

7. Dérivé de désoxy-2 ribonucléoside selon la revendication 2, caractérisé en ce qu'il est constitué par la [N amido-1]uréido-3' désoxy-3' thymidine.

8. Dérivé de désoxy-2 ribonucléoside selon la revendication 3, caractérisé en ce qu'il est constitué par la S-(N,N-diéthylthiocarbamoyl)-3' désoxy-3' thio-3' thymidine.

9. Dérivé de désoxy-2 ribonucléoside selon la revendication 3, caractérisé en ce qu'il est constitué par l'O-(N, N'-diméthylthiocarbamoyl)-3' thymidine.

10. Dérivé de désoxy-2 ribonucléoside selon la revendication 3, caractérisé en ce qu'il est constitué par l'O-(imidazolothiocarbamoyl)-3' thymidine.

11. Dérivé de désoxy-2 ribonucléoside selon la revendication 3, caractérisé en ce qu'il est constitué par l'O-(morpholinothiocarbamoyl)-3' thymidine.

12. Dérivé de désoxy-2 ribonucléoside selon la revendication 4, caractérisé en ce qu'il est constitué par la dicyanamido-3' désoxy-3' thymidine.

13. Médicament pouvant être, notamment, utilisé comme agent antibiotique, antiviral, antitumoral et immunomodulateur, caractérisé en ce qu'il contient, à titre de principe actif, un dérivé de désoxy-2 ribonucléoside défini dans l'une des revendications 1 à 12.

Revendications pour l'Etat contractant suivant:GR

1. Dérivé de désoxy-2 ribonucléoside caractérisé en ce qu il répond à la formule générale

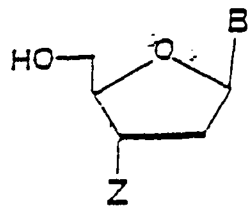

(I)

dans laquelle B désigne une base pyrimidique telle que la thymine ou l'uracyle ou un dérivé équivalent et Z et un groupement
A- C -X
‖
Y
ou encore un groupement $N(CN)_2$ ou $N(CONH_2)_2$ où A représente un radical alkyle saturé ou non, substitué ou non par des groupements carbonés ou des hétéroatomes, ou un groupe aminé primaire, secondaire ou tertiaire, ou encore un cycle insaturé ou non, comportant ou non des hétéroatomes, X représente un atome d'azote, d'oxygène ou de soufre ou encore de sélénium, lié au carbone du cycle glucidique, et Y représente un atome d'azote, d'oxygène, de soufre ou de sélénium, à l'exclusion des dichlorhydrates de O-[-ω-[N-[ω-(diéthylamino) alkyl] diéthylammonio]-acyl-]-3′ thymidine, des esters du N,N′-di-n-alkyldiabicyclo-1,4 octane [2.2.2.] et du O-[Phénoxy(thiocarbonyl)]-3′ -desoxy-2′-thymidine.

2. Dérivé de désoxy-2 ribonucléoside caractérisé en ce qu'il répond à la formule générale donnée dans la revendication 1, dans laquelle B a la signification précitée et Z représente un groupement
A- C -X
‖
Y
où X et Y ont la signification précitée et A représente un radical alkyle inférieur contenant 1 à 8 atomes de carbone substitué ou non par un ou plusieurs atomes d'halogène, et plus particulièrement par des atomes de fluor.

3. Dérivé de désoxy-2 ribonucléoside selon la revendication 1, caractérisé en ce qu'il répond à la formule générale donnée dans la revendication 1, dans laquelle B a la signification précitée et Z représente un groupement
A- C-X
‖
Y
où X et Y ont la signification précitée et A représente un groupement diméthylamino, diéthylamino, isopropylamino, diisopropylamino, morpholino et imidazolo.

4. Dérivé de désoxy-2 ribonucléoside selon la revendication 1, caractérisé en ce qu'il répond à la formule générale donnée dans la revendication 1 dans laquelle B a la signification précitée et Z représente un groupement N ≡ C-X où X représente l'azote.

5. Dérivé de désoxy-2 ribonucléoside selon la revendication 2, caractérisé en ce qu'il est constitué par la O-butanoyl-3′ thymidine.

6. Dérivé de désoxy-2 ribonucléoside selon la revendication 2, caractérisé en ce qu'il est constitué par l'O-heptafluorobutanoyl-3′ thymidine.

7. Dérivé de désoxy-2 ribonucléoside selon la revendication 2, caractérisé en ce qu'il est constitué par la [N amido-1]uréido-3′ désoxy-3′ thymidine.

8. Dérivé de désoxy-2 ribonucléoside selon la revendication 3, caractérisé en ce qu'il est constitué par la S-(N,N-diéthylthiocarbamoyl)-3′ désoxy-3′ thio-3′ thymidine.

9. Dérivé de désoxy-2 ribonucléoside selon la revendication 3, caractérisé en ce qu'il est constitué par l'O-(N, N'-diméthylthiocarbamoyl)-3′ thymidine.

10. Dérivé de désoxy-2 ribonucléoside selon la revendication 3, caractérisé en ce qu'il est constitué par l'O-(imidazolothiocarbamoyl)-3′ thymidine.

11. Dérivé de désoxy-2 ribonucléoside selon la revendication 3, caractérisé en ce qu'il est constitué par l'O-(morpholinothiocarbamoyl)-3′ thymidine.

12. Dérivé de désoxy-2 ribonucléoside selon la revendication 4, caractérisé en ce qu'il est constitué par la dicyanamido-3′ désoxy-3′ thymidine.